(19) Europäisches Patentamt
European Patent Office
Office européen des brevets

(11) Publication number: **0 180 854**
**B1**

(12) # EUROPEAN PATENT SPECIFICATION

(45) Date of publication of patent specification: **22.06.88**

(51) Int. Cl.$^4$: **C 07 C 79/18,** A 01 N 37/02

(21) Application number: **85113474.2**

(22) Date of filing: **23.10.85**

(54) **Chloroacetate derivatives and germicides.**

(30) Priority: **09.11.84 JP 235103/84**

(43) Date of publication of application:
**14.05.86 Bulletin 86/20**

(45) Publication of the grant of the patent:
**22.06.88 Bulletin 88/25**

(84) Designated Contracting States:
**DE FR GB IT NL**

(56) References cited:

**No relevant documents haven been disclosed**

(73) Proprietor: **HODOGAYA CHEMICAL CO., LTD.**
**4-2, Toranomon 1-chome Minato-ku**
**Tokyo (JP)**

(72) Inventor: **Kawada, Hiroshi**
**1049-50, Miyawada Fujishiro-cho**
**Kitasoma-gun Ibaragi-ken (JP)**
Inventor: **Shindo, Noboru**
**8-6, Higashi-Tamagawagakuen 1-chome**
**Machida-shi Tokyo (JP)**

(74) Representative: **Wächtershäuser, Günter, Dr.**
**Tal 29**
**D-8000 München 2 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 180 854 B1**

**Description**

The present invention relates to a 2-nitroalkyl chloroacetate and a germicide containing it as an active ingredient.

In recent years, intensive horticultural cultivation and cultivation of garden products have become popular. Accordingly, not only the efficiency in cultivation but also repeated cultivation on the same ground is required. In general, repeated cultivation of the same crop on the same ground tends to lead to a decrease in the harvest without application of agricultural chemicals, and in an extreme case, there will be substantially no harvest. It is believed that this is caused mainly by soil disease-germs. In order to avoid such an undesirable situation, it has been common to employ gaseous fumigating agents such as chloropicrin, methyl bromide or methyl isothiocyanate. However, such agents have some drawbacks. For instance, 1) they show strong phytotoxicity against crop plants, 2) they bring about an inconvenience in their application such that after their application to the soil surface to attain the desired effectiveness, it is necessary to plow the soil to prevent phytotoxicity, and 3) some of them are lachrymatory or skin-irritative, and some others are likely to induce mutation, thus bringing about a difficulty in the safety. Under the circumstances, it has been desired to develop an effective agent which has no phytotoxicty and which is convenient and safe in its use.

The present inventors have conducted extensive studies to solve such problems, and have finally accomplished the present invention on the basis of a discovery that certain chloroacetate compounds have extremely effective germicidal activities.

In a first aspect, the present invention provides a compound having the formula:

$$ClCH_2COO\underset{R_1}{\underset{|}{CH}}\underset{R_2}{\underset{|}{CH}}NO_2 \qquad (I)$$

wherein each of $R_1$ and $R^2$ is a hydrogen atom or a lower alkyl group.

In a second aspect, the present invention provides a germicide comprising a germicidally effective amount of the compound of the formula I and a carrier.

Now, the present invention will be described in further detail with reference to the preferred embodiments.

The compounds of the present invention are readily synthesized by the following methods.

Namely, they can be synthesized almost quantitatively in a simple manner by such a method in which a 2-nitroalkyl alcohol and chloroacetic acid chloride or chloroacetic acid anhydride are reacted in the presence of a base, or a method in which a 2-nitroalkyl alcohol and chloroacetic acid are condensed by means of a dehydrating agent.

The physical properties of representative compounds of the present invention are as shown in Table 1.

TABLE 1

| Compound No. | Boiling point (°C/mmHg) | CMR (ppm) | | |
|---|---|---|---|---|
| No.1 | 53—55/0.1 | 40.5(t) | 60.9(t) | |
| $R^1$:H | | 73.2(t) | 166.8(s) | |
| $R^2$:H | | | | |
| No. 2 | 61—63/0.09 | 15.3(q) | 40.3(t) | 65.4(t) |
| $R^1$:H | | 80.5(d) | 166.4(s) | |
| $R^2$:$CH_3$ | | | | |
| No. 3 | 72—73/0.1 | 9.5(q) | 23.3(t) | 40.2(t) |
| $R^1$:H | | 64.5(t) | 87.0(d) | 166.6(s) |
| $R^2$:$C_2H_5$ | | | | |
| No. 4 | 62—63/0.1 | 16.1(q) | 40.2(t) | 68.3(d) |
| $R^1$:$CH_3$ | | 77.4(t) | 165.8(s) | |
| $R^2$:H | | | | |
| No. 5 | 70—71/0.08 | 8.8(q) | 24.2(t) | 40.5(t) |
| $R^1$:$C_2H_5$ | | 72.7(d) | 76.5(t) | 166.3(s) |
| $R^2$:H | | | | |
| No. 6 | 69—72/0.07 | 12.9(q) | 15.5(q) | 40.5(d) |
| $R^1$:$CH_3$ | | 71.5(d) | 72.3(d) | |
| $R^2$:$CH_3$ | | 84.0(d) | 85.2(d) | 165.9(s) |

Compound No. 1: $ClCH_2COOCH_2CH_2NO_2$
Compound No. 2: $ClCH_2COOCH_2CH(CH_3)NO_2$
Compound No. 3: $ClCH_2COOCH_2CH(C_2H_5)NO_2$
Compound No. 4: $ClCH_2COOCH(CH_3)CH_2NO_2$
Compound No. 5: $ClCH_2COOCH(C_2H_5)CH_2NO_2$
Compound No. 6: $ClCH_2COOCH(CH_3)CH(CH_3)NO_2$

The compounds of the present invention are useful not only for the agricultural purposes, but also in many other fields such as medical or industrial fields wherein microorganisms are concerned.

Further, they may be used in various optional formulations such as oil, emulsifiable concentrates, wettable powders, granules or dusts by mixing them with various carriers depending upon the particular purposes. Particularly useful are emulsifiable concentrates and granules. The carriers for this purpose may be liquid or solid, or a combination thereof. As solid carriers, there may be mentioned bentonite, talc, clay, diatomaceous earth, fuller's earth (Japanese acid clay) or dextrin. As liquid carriers, there may be mentioned inert solvents such as xylene, isophorone, kerosine or alcohols.

For the preparation of formulations, surfactants or the like may be incorporated to provide emulsifiable or dispersible properties. Such formulations are readily obtainable by conventional methods for the preparation of agricultural chemicals which are commonly employed.

The effective amount of the active ingredient is usually within a range of from 5 to 30% by weight in the case of granules, and within a range of from 5 to 95% by weight in the case of oil or emulsifiable concentrates. The compounds of the present invention may be used alone. However, in some cases, they may be used in a combination with other agricultural chemicals such as other germicides, insecticides or herbicides. Compounds of the present invention have no phytotoxicity, and they are simple in use and show little toxicity against human and animals and extremely high germicidal activities.

The compounds of the present invention are effective against Gram positive bacteria, Gram negative bacteria, Ascomycetes, Basidomycetes, Phycomycetes, etc.

Now, the effectiveness of the compounds of the present invention will be shown by Test Examples.

Test Example 1

To a 20 ml of a meat extract-peptone agar culture medium, 100 µl of a dimethylsulfoxide solution of a 2-nitroalkyl chloroacetate having a predetermined concentration was added, and the mixture was poured into a Petri dish to form a plate culture medium. Onto this culture medium, bacillus subtilis and Salmonella typhimurium were applied in straight lines and cultured at 37°C overnight, whereupon the concentration for the inhibition of the growth of the bacteria was determined. The evaluation of the effectiveness was made in accordance with the following ratings:

| Ratings | Evaluation standards |
|---|---|
| 4 | Complete growth inhibition |
| 3 | More than 75% growth inhibition |
| 2 | 50—75% growth inhibition |
| 1 | 25—50% growth inhibition |
| 0 | No substantial growth inhibition |

The results are shown in Table 2

TABLE 2

| Bacteria | Bacillus subtilis | | | | Salmonella typhimurium | | | |
|---|---|---|---|---|---|---|---|---|
| Concentration | Concentration (ppm) | | | | Concentration (ppm) | | | |
| Compound No. | 12.5 | 25 | 50 | 100 | 12.5 | 25 | 50 | 100 |
| No. 1 | 0 | 0 | 0 | 0 | 0 | 0 | 0 | 0 |
| No. 2 | 0 | 1 | 4 | 4 | 0 | 3 | 4 | 4 |
| No. 3 | 0 | 0 | 4 | 4 | 0 | 0 | 4 | 4 |
| No. 4 | 0 | 0 | 0 | 0 | 0 | 0 | 0 | 0 |
| No. 5 | 0 | 0 | 0 | 0 | 0 | 0 | 0 | 2 |
| No. 6 | 0 | 0 | 4 | 4 | 0 | 3 | 4 | 4 |

Test Example 2

To 20 ml of a potato-dextrose agar culture medium (PDA medium), 100 µl of a dimethylsulfoxide solution of a 2-nitroalkyl chloroacetate having a predetermined concentration was added at a temperature of from 40 to 42°C, and the mixture was poured into a Petri dish to form a plate culture medium. Onto this culture medium, two disc specimens with a diameter of 4 mm of each of Fusarium solani, Rhizoctonia solani and Pythium aphanidermatum, which were preliminarily cultured on PDA culture media, were placed, and cultured at 25°C for 5 days in the case of Fusarium solani, and 3 days in the cases of Rhizoctonia solani and Pythium aphanidermatum. Then, the diameter of each colony was measured, and the inhibition value was calculated as compared with the non-treated medium, and thus the effectiveness was evaluated.

$$\text{Inhibition value} = \left(1 - \frac{\text{Colony diameter on treated medium}}{\text{Colony diameter on non-treated medium}}\right) \times 100$$

The test was repeated twice for each concentration, and to the non-treated medium, only dimethylsulfoxide was added. The average values of the results are shown in Table 3.

TABLE 3

| Bacteria | Pythium aphanidermatum | | | Fusarium solani | | | Rhizoctonia solani | | |
|---|---|---|---|---|---|---|---|---|---|
| Concentration | Concentration (ppm) | | | | | | | | |
| Compound No. | 12.5 | 25 | 50 | 12.5 | 25 | 50 | 3.125 | 6.25 | 12.5 |
| No. 1 | 16 | 50 | 92 | 20 | 100 | 100 | 16 | 77 | 100 |
| No. 2 | 31 | 74 | 100 | 11 | 100 | 100 | 16 | 100 | 100 |
| No. 3 | 41 | 71 | 93 | 9 | 18 | 59 | 9 | 29 | 87 |
| No. 4 | 14 | 39 | 92 | 14 | 72 | 100 | 0 | 7 | 64 |
| No. 5 | 21 | 64 | 94 | 16 | 41 | 100 | 6 | 23 | 40 |
| No. 6 | 67 | 94 | 100 | 22 | 40 | 100 | 28 | 53 | 75 |

Test Example 3

Into a 1/10,000a pot, 1 kg of soil infested with Fusarium oxysporum raphani, was put, and one of the compounds of the present invention according to Example 1 given hereinafter and comparative agents i.e. Difolatan [N-(1,1,2,2-tetrachloroethylthio)-4-cyclohexene-1,2-dicarboxyimide] and chloropicrin, was mixed to the soil in a predetermined amount. The treated soil was left to stand in a glass chamber (20—30°C) for a week. Further, in the case of chloropicrin, the soil was spread for degassing for 3 hours. Then, 15 seeds per pot of a Japanese radish (variety: Aokubimiyashige) were sown. Three weeks after the seeding, the presence or absence of the blackening of the stem portion was examined to determine the deseased rate. The test was repeated twice for each compound. The average values of the results are shown in Table 4.

TABLE 4

| Compound No. | Concentration of the active ingredient (ppm) | Diseased rate (%) | Phytotoxicity |
|---|---|---|---|
| No. 1 | 50 | 0 | Nil |
| | 100 | 0 | Slight growth inhibition |
| No. 2 | 50 | 16 | Nil |
| | 100 | 0 | Nil |
| No. 3 | 50 | 8 | Nil |
| | 100 | 6 | Nil |
| No. 4 | 50 | 6 | Nil |
| | 100 | 0 | Slight rolling of leaves |
| No. 5 | 50 | 8 | Nil |
| | 100 | 0 | Nil |
| No. 6 | 50 | 16 | Nil |
| | 100 | 0 | Nil |
| (Comparative compounds) | | | |
| Diafolatan | 50 | 17 | Slight growth inhibition |
| Chloropicrin | 300 | 3 | Nil |
| Non-treatment | — | 88 | Nil |

Test Example 4

At an agricultural field infested with Fusarium oxysporum f. Sp. raphani, an oil agent of 2-nitroethyl chloroacetate according to Example 5 given hereinafter, was applied to the soil surface at a rate of 40 liter per 10 acres, and immediately, the soil was plowed by a tractor. As a comparative agent, chloropicrin was applied by injection treatment at 30 cm intervals at a depth of 15 cm from the soil surface at a rate of 20 liter per 10 acres, and 7 days later, the soil was plowed for degassing. Ten days after the application of the germicide, seeds of a Japanese radish (variety: Aokubimiyashige) were sown at 30 cm intervals. One month later, thinning out was conducted, and 3 months later, roots were cut off to examine the presence or absence of the disease. The test was repeated 3 times each with a unit field area of 7.5 $m^2$, and 40 radishes per unit field area were examined. The average values of the results are shown in Table 5.

TABLE 5

| Germicide | Amount of the active ingredient applied (kg/10 a) | Diseased rate |
|---|---|---|
| Germicide of the present invention | 19.2 | 5.8% |
| Chloropicrin | 32.3 | 6.7% |
| Non-treatment | — | 81.7% |

As shown above, 2-nitroalkyl chloroacetates of the present invention show high germicidal activities against not only various filamentous fungi which give serious damages to crops, but also Gram positive bacteria and Gram negative bacteria which create various problems in various fields. Yet, they do not require cumbersome degassification as is required in the case of chloropicrin, and they show no substantial phytotoxicity to crop plants.

Now, formulation examples of the present invention will be described. In these Examples, "parts" means "parts by weight".

Example 1: Granules

| | |
|---|---|
| 2-Nitroethyl chloroacetate | 20 parts |
| Granular terra abla | 80 parts |

The active ingredient was impregnated in granular terra abla to obtain the granules of the present invention.

Example 2: Granules

| | |
|---|---|
| Bentonite | 35 parts |
| Diatomaceous earth | 60 parts |
| Nonaryl 204 (trademark, manufactured by Toho Kagaku K.K.) | 2 parts |
| Rapizol B-80 (trademark, manufactured by Nippon oil and Fat Co., Ltd.) | 3 parts |

The above components were mixed and kneaded with water. The mixture was granulated in a wet system, and then dried. To 70 parts of the dried granular product, 30 parts of 2-nitropropyl chloroacetate was impregnated to obtain granules of the present invention.

Example 3: Emulsion

| | |
|---|---|
| 2-Nitrobutyl chloroacetate | 85 parts |
| Xylene | 12 parts |
| Sorpol 900B (trademark, manufactured by Toho Kagaku K.K.) | 3 parts |

The above components were mixed to obtain an emulsion of the present invention.

Example 4: Oil

| | |
|---|---|
| 1-Methyl-2-nitroethyl chloroacetate | 50 parts |
| Xylene | 50 parts |

The above components were mixed to obtain an oil of the present invention.

Example 5: Oil

| | |
|---|---|
| 2-Nitroethyl chloroacetate | 40 parts |
| 2-Chlorotoluene | 60 parts |

The above components were mixed to obtain an oil of the present invention.

Example 6: Emulsifiable concentrate

| | |
|---|---|
| 2-Nitroethyl chloroacetate | 50 parts |
| Xylene | 40 parts |
| Toxanon 1006 (trademark, manufactured by Sanyo Chemical Industries Co., Ltd.) | 10 parts |

The above components were mixed to obtain an emulsion of the present invention.

**Claims**

1. A 2-nitroalkyl chloroacetate derivative having the formula:

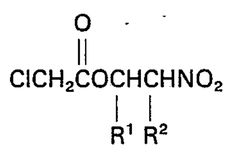

(I)

wherein each of $R^1$ and $R^2$ is a hydrogen atom or a lower alkyl group.

2. The 2-nitroalkyl chloroacetate derivative according to Claim 1, wherein the lower alkyl group is a methyl group or an ethyl group.

3. 2-Nitroethyl chloroacetate according to Claim 1.

4. 1-Methyl-2-nitroethyl chloroacetate according to Claim 1.

5. 1-Ethyl-2-nitroethyl chloroacetate according to Claim 1.

6. 2-Nitropropyl chloroacetate according to Claim 1.

7. 2-Nitrobutyl chloroacetate according to Claim 1.

8. 1-Methyl-2-nitropropyl chloroacetate according to Claim 1.

9. A germicide comprising a germicidally effective amount of a 2-nitroalkyl chloroacetate derivative as defined in Claim 1 and a carrier.

**Patentansprüche**

1. 2-Nitroalkyl-chloracetatderivat der Formel

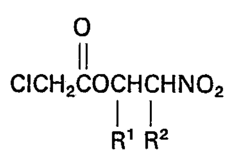

(I)

wobei jeder der Reste $R^1$ und $R^2$ ein Wasserstoffatom oder eine niedere Alkylgruppe ist.

2. 2-Nitroalkyl-chloracetat derivat gemäß Anspruch 1, wobei die niedrige Alkylgruppe eine Methyl- oder eine Äthylgruppe ist.

3. 2-Nitroäthylchloracetat gemäß Anspruch 1.

4. 1-Methyl-2-nitroäthylchloracetat gemäß Anspruch 1.

5. 1-Äthyl-2-nitroäthylchloracetate gemäß Anspruch 1.
6. 2-Nitropropylchloracetat gemäß Anspruch 1.
7. 2-Nitrobutylchloracetat gemäß Anspruch 1.
8. 1-Methyl-2-nitropropylchloracetat gemäß Anspruch 1.
9. Germizides Mittel, umfassend eine germizid wirksame Menge eines 2-Nitroalkylchloracetatderivats, wie es in Anspruch 1 definiert ist, und einen Träger.

**Revendications**

1. Chloracétate de nitro-2 alkyle ayant la formule:

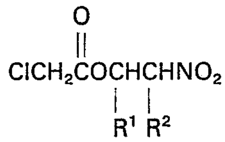

(I)

dans laquelle $R^1$ et $R^2$ représentent chacun un atome d'hydrogène ou un groupe alkyl inférieur.
2. Chloracétate de nitro-2 alkyle selon la revendication 1, dans lequel le groupe alkyle inférieur est un groupe méthyle ou un groupe éthyle.
3. Chloracétate de nitro-2 éthyle, selon la revendication 1.
4. Chloracétate de méthyl-1 nitro-2 éthyle, selon la revendication 1.
5. Chloracétate d'éthyl-1 nitro-2 éthyle, selon la revendication 1.
6. Chloracétate de nitro-2 propyle, selon la revendication 1.
7. Chloracétate de nitro-2 butyle, selon la revendication 1.
8. Chloracétate de méthyl-1 nitro-2 propyle, selon la revendication 1.
9. Germicide comprenant une quantité ayant une efficacité germicide, du chloracétate de nitro-2 alkyle, tel que défini à la revendication 1, et un support.